# EUROPEAN PATENT APPLICATION

(11) **EP 3 845 958 A1**
(43) Date of publication of application: **07.07.2021**
(21) Application number: 19856377.7
(22) Date of filing: 26.08.2019
(51) Int. Cl.: G02F 1/13, A61B 8/00, G02F 1/1335

(54) **ULTRASONIC DIAGNOSTIC APPARATUS AND LIQUID CRYSTAL DISPLAY DEVICE**

(30) Priority: 30.08.2018 JP 2018162114
(71) Applicant: Sony Corporation, 108-0075 Tokyo (JP)
(72) Inventor: HATA, Kenji, Tokyo 108-0075 (JP); HAYASHIMOTO, Seiji, Tokyo 108-0075 (JP)
(74) Representative: D Young & Co LLP
(86) International application number: PCT/JP2019/033397
(87) International publication number: WO 2020/045374

(57) **Abstract**

An ultrasound diagnostic apparatus (200) is provided that includes: a probe (210) that receives an ultrasound signal reflected off an object; a processing unit (260) that generates image information based on the ultrasound signal; and a liquid crystal display (270) that displays the image information, wherein the liquid crystal display includes: a liquid crystal panel (110) including a pair of substrates (113, 114) arranged in an opposing manner and a liquid crystal layer (115) sandwiched between the pair of substrates; a pair of polarizing plates (111, 112) arranged on a light source side and a display surface side, respectively, of the panel and forming crossed Nicol prism; a first optical element (120) in which a light-blocking layer and a light-transmitting layer are alternately and continuously arranged in a planar direction, the first optical element (120) being arranged between the polarizing plate on the light source side and the light source; and a second optical element (130) having a surface scattering function arranged on the polarizing plate on the display surface side, and the first optical element is arranged such that a major axis of the light-blocking layer that is an axis extending in a light-transmitting direction is oriented in a direction determined based on at least one of the crossed Nicol prism and the second optical element.

## Description

### [Technical Field]

The present disclosure relates to an ultrasound diagnostic apparatus and a liquid crystal display apparatus.

### [Background Art]

The contrast ratio of a display is an index that is important for appropriately identifying an object to be observed. For example, apparatuses required to have a high contrast ratio in the medical field include ultrasound diagnostic apparatuses that irradiate an object with an ultrasound signal and generate image information based on the ultrasound signal reflected off the object. Not limited to the ultrasound diagnostic apparatuses, there is a tendency that a high contrast ratio is required for image information used in the medical field (needless to say, this tendency is not limited to the medical field). As displays capable of realizing a high contrast ratio, for example, organic EL (ElectroLuminescence) displays, in which OLEDs (Organic Light-Emitting Diodes) are used as light-emitting elements, have been developed. Since the OLEDs are spontaneous light-emitting elements, the organic EL displays can appropriately express black by causing the OLEDs not to emit light, and can realize a high contrast ratio. For example, some ultrasound diagnostic apparatuses use the organic EL displays. However, the manufacturing costs of the organic EL displays tend to be relatively high.

Meanwhile, the liquid crystal displays are cheaper than the organic EL displays in terms of the manufacturing costs, but cannot completely prevent light leakage due to the structure in which black is expressed by interrupting light from a backlight using the orientation of liquid crystal molecules. Accordingly, the liquid crystal displays tend to be inferior to the organic EL displays in terms of the contrast ratio.

In recent years, techniques for improving the contrast ratio of the liquid crystal displays have been actively developed. For example, PTL 1 below discloses a technique that makes it possible to improve the viewing angle and the contrast ratio by providing a viewing angle improving sheet and a sheet in which a fine louver is incorporated.

### [Citation List]

### [Patent Literature]

[PTL 1] JP H9-179107A

### [Summary]

### [Technical Problem]

However, there have been cases where the contrast ratio is not sufficiently improved by the technique disclosed in PTL 1 or the like. For example, there are cases where the contrast ratio is not sufficiently improved due to the louver being not appropriately arranged with respect to a crossed Nicol prism formed by a pair of polarizing plates arranged respectively on a light source side and a display surface side of a liquid crystal panel, or the viewing angle improving sheet.

The present disclosure has been made in view of the foregoing, and provides a novel and improved ultrasound diagnostic apparatus and liquid crystal display apparatus that are capable of appropriately improving the contrast ratio.

### [Solution to Problem]

According to the present disclosure, an ultrasound diagnostic apparatus is provided that includes: a probe that irradiates an object with an ultrasound signal and receives an ultrasound signal reflected off the object; a processing unit that generates image information based on the reflected ultrasound signal; and a liquid crystal display that displays the image information, wherein the liquid crystal display includes: a liquid crystal panel including a pair of substrates arranged in an opposing manner, and a liquid crystal layer sandwiched between the pair of substrates; a pair of polarizing plates arranged on a light source side and a display surface side, respectively, of the liquid crystal panel, and forming a crossed Nicol prism; a first optical element in which a light-blocking layer and a light-transmitting layer are alternately and continuously arranged in a planar direction, the first optical element being arranged between the polarizing plate on the light source side and the light source; and a second optical element having a surface scattering function and arranged on the polarizing plate on the display surface side, and the first optical element is arranged such that a major axis of the light-blocking layer that is an axis extending in a direction in which light is transmitted is oriented in a direction determined based on at least one of the crossed Nicol prism and the second optical element.

Also, according to the present disclosure, a liquid crystal display apparatus is provided that includes: a liquid crystal panel including a pair of substrates arranged in an opposing manner, and a liquid crystal layer sandwiched between the pair of substrates; a pair of polarizing plates arranged on a light source side and a display surface side, respectively, of the liquid crystal panel, and forming a crossed Nicol prism; a first optical element in which a light-blocking layer and a light-transmitting layer are alternately and continuously arranged in a planar direction, the first optical element being arranged between the polarizing plate on the light source side and the light source; and a second optical element having a surface scattering function and arranged on the polarizing plate on the display surface side, wherein the first optical element is arranged such that a major axis of the light-blocking layer that is an axis extending in a direction in which light is transmitted is oriented in a direction determined based on at least one of the crossed Nicol prism and the second optical element.

### [Advantageous Effects of Invention]

As described above, according to the present disclosure, the contrast ratio can be improved more appropriately.

Note that the above effect is not necessarily limited, and any of the effects described in the present specification or other effects that may be understood from the present specification may be achieved together with the above effect, or in place of the above effect.

### [Brief Description of Drawings]

[Fig. 1]

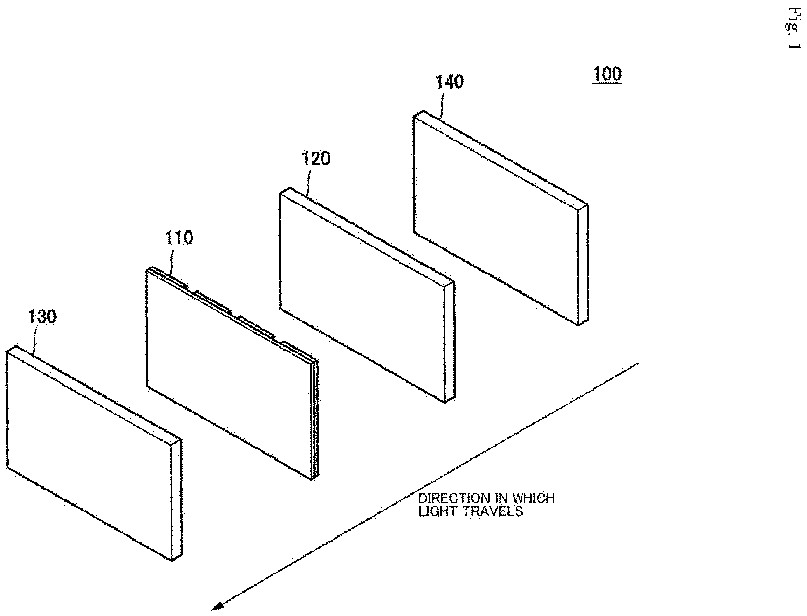

   Fig. 1 is a diagram showing a configuration of an entire liquid crystal display apparatus 100.
[Fig. 2]

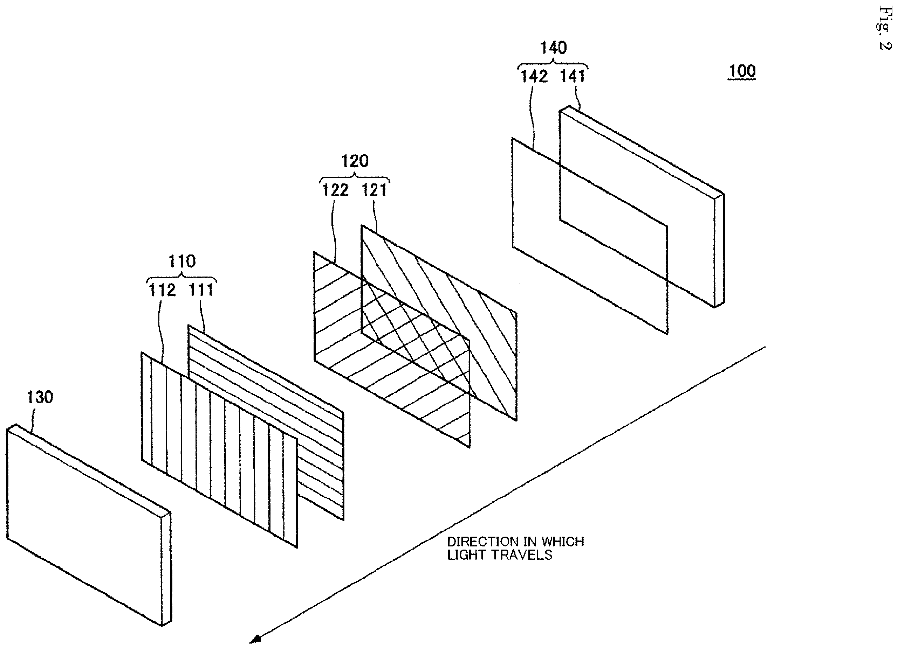

   Fig. 2 is a diagram showing a configuration of the entire liquid crystal display apparatus 100.
[Fig. 3]

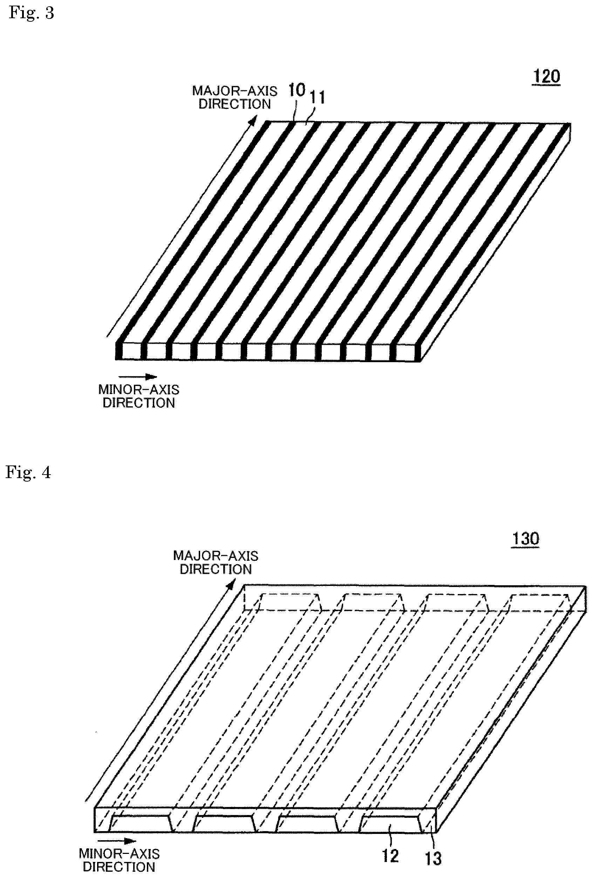

   Fig. 3 is a conceptual diagram of a condensing film 120.
[Fig. 4]
   Fig. 4 is a conceptual diagram of a viewing angle control film 130.
[Fig. 5]

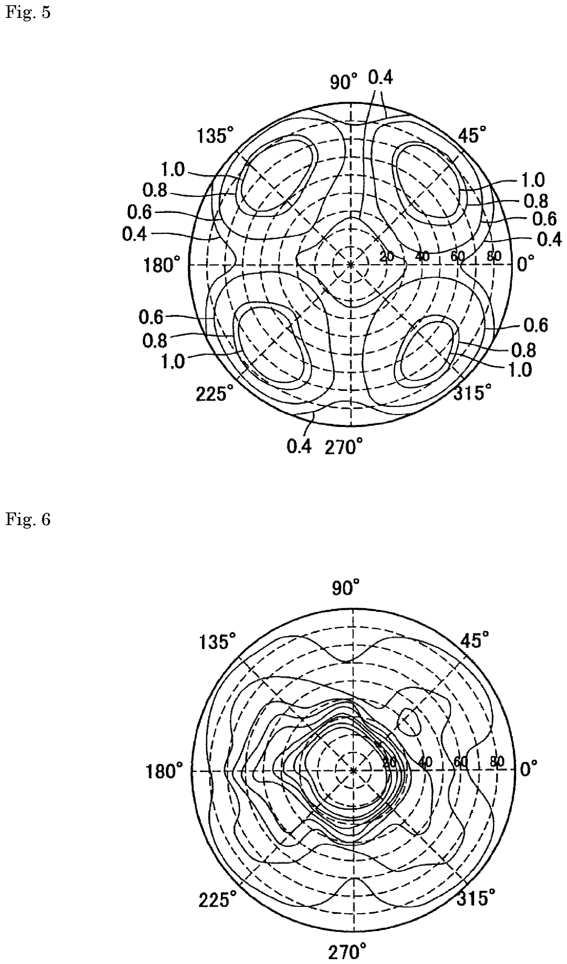

   Fig. 5 is a diagram showing measured values of the viewing angle for black brightness before the present disclosure is applied.
[Fig. 6]
   Fig. 6 is a diagram showing measured values of the viewing angle for black brightness after the present disclosure is applied.
[Fig. 7]

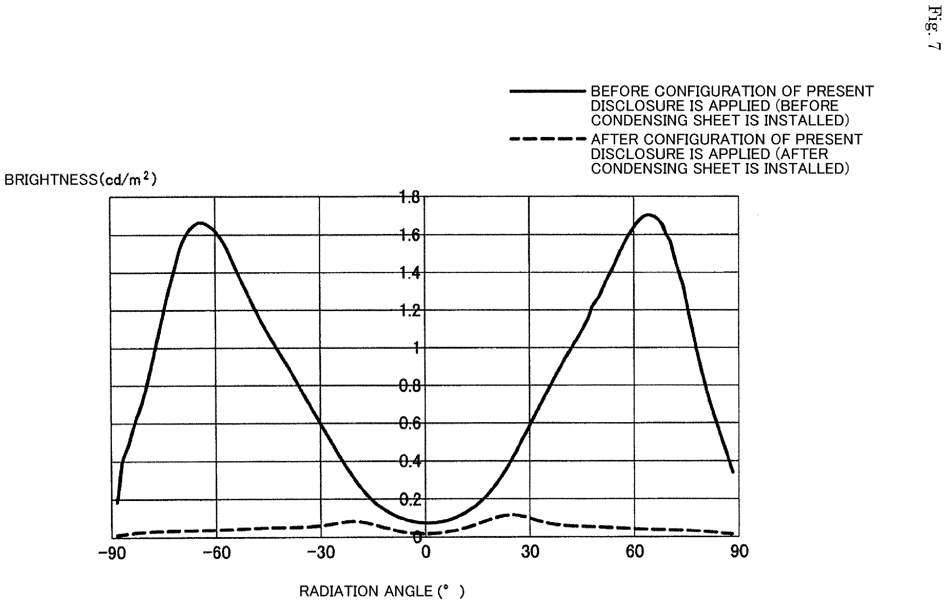

   Fig. 7 is a diagram showing measured values of the viewing angle for black brightness at an azimuth 45° (and 225°) before and after the present disclosure is applied.
[Fig. 8]

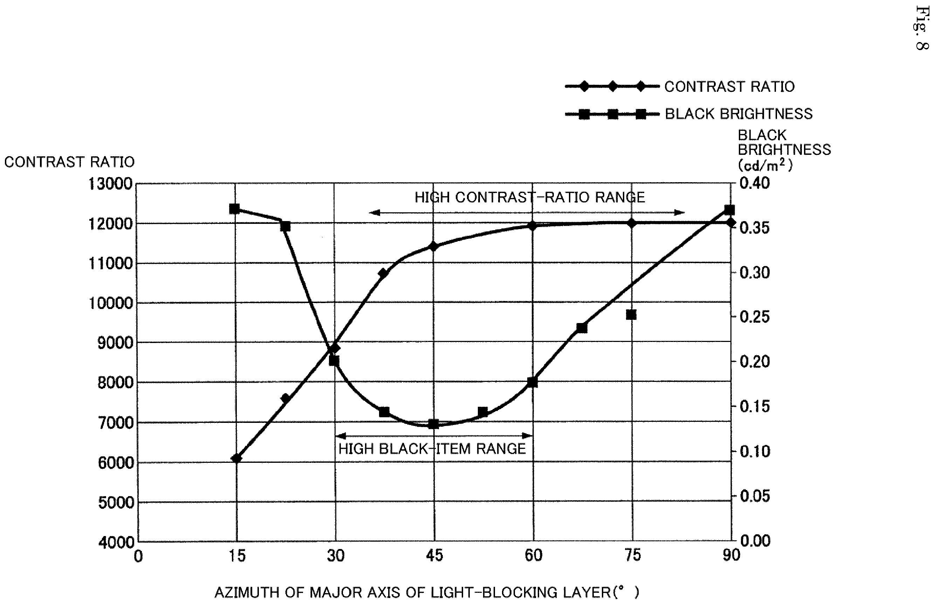

   Fig. 8 shows the relationship between the azimuth of a major axis of a light-blocking layer 10 in the condensing film 120, the black brightness, and the contrast ratio.
[Fig. 9]

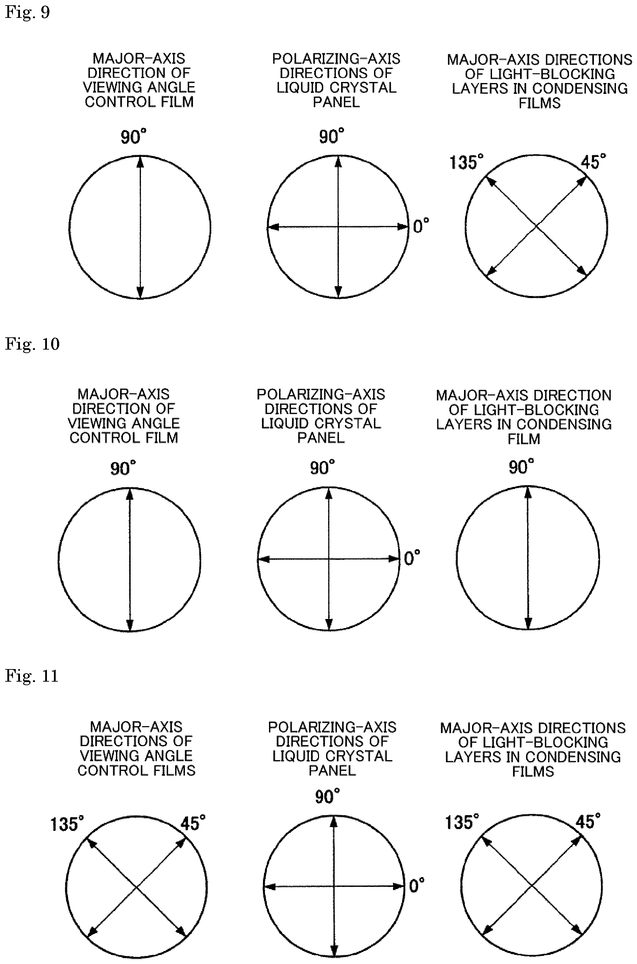

   Fig. 9 is a diagram showing a variation of the installation mode of a liquid crystal panel 110, the condensing film 120, and the viewing angle control film 130.
[Fig. 10]
   Fig. 10 is a diagram showing a variation of the installation mode of the liquid crystal panel 110, the condensing film 120, and the viewing angle control film 130.
[Fig. 11]
   Fig. 11 is a diagram showing a variation of the installation mode of the liquid crystal panel 110, the condensing film 120, and the viewing angle control film 130.
[Fig. 12]

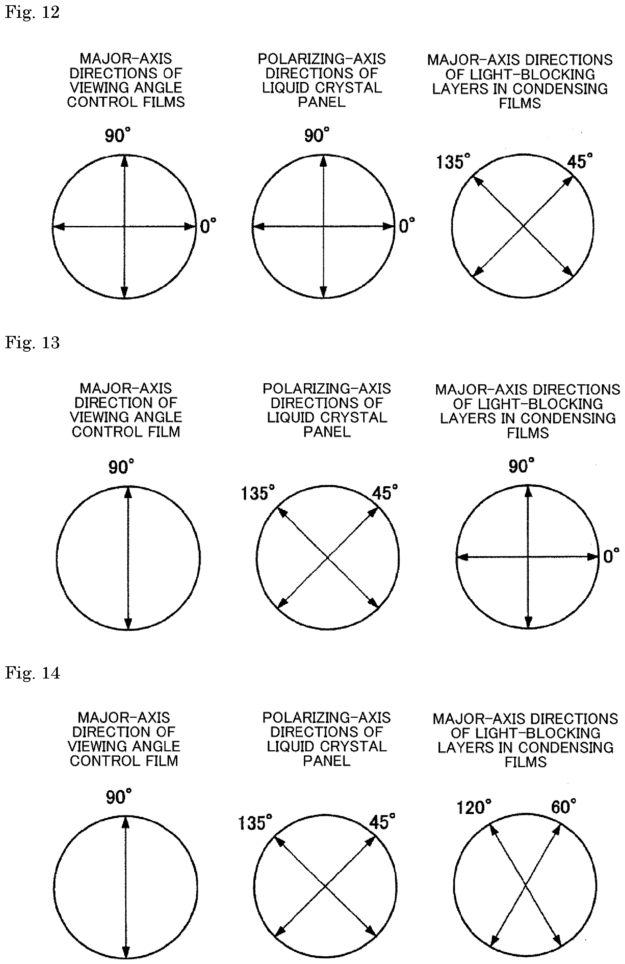

   Fig. 12 is a diagram showing a variation of the installation mode of the liquid crystal panel 110, the condensing film 120, and the viewing angle control film 130.
[Fig. 13]
   Fig. 13 is a diagram showing a variation of the installation mode of the liquid crystal panel 110, the condensing film 120, and the viewing angle control film 130.
[Fig. 14]
   Fig. 14 is a diagram showing a variation of the installation mode of the liquid crystal panel 110, the condensing film 120, and the viewing angle control film 130.
[Fig. 15]

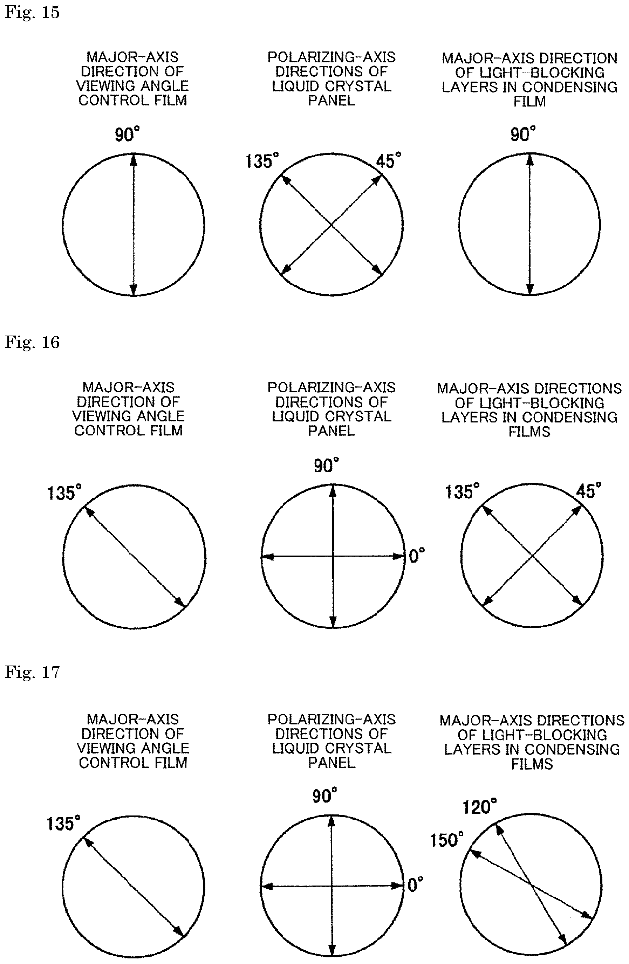

   Fig. 15 is a diagram showing a variation of the installation mode of the liquid crystal panel 110, the condensing film 120, and the viewing angle control film 130.
[Fig. 16]
   Fig. 16 is a diagram showing a variation of the installation mode of the liquid crystal panel 110, the condensing film 120, and the viewing angle control film 130.
[Fig. 17]
   Fig. 17 is a diagram showing a variation of the installation mode of the liquid crystal panel 110, the condensing film 120, and the viewing angle control film 130.
[Fig. 18]

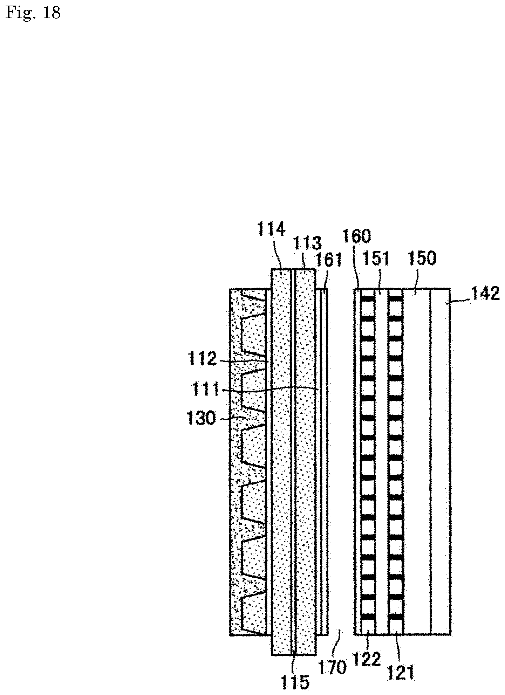

   Fig. 18 is a diagram for illustrating a method for fixing components.
[Fig. 19]

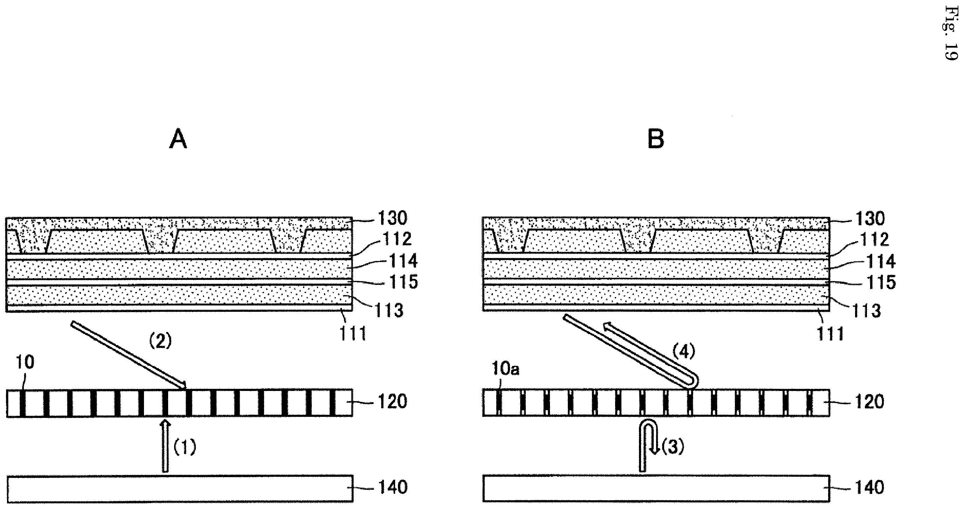

   Fig. 19 is a diagram for illustrating use of the condensing film 120 that partially has a reflection structure.
[Fig. 20]

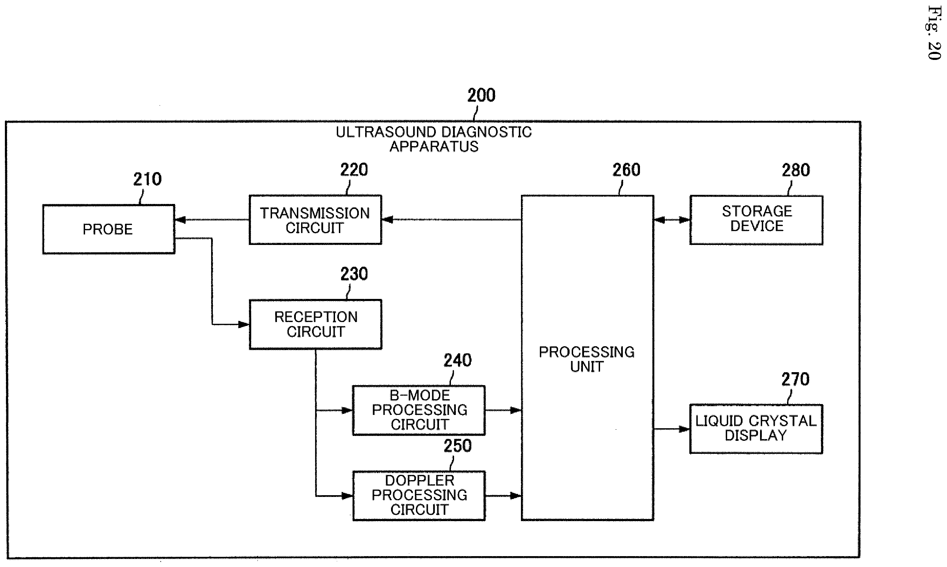

   Fig. 20 is a block diagram showing an example configuration of an ultrasound diagnostic apparatus 200 in the case where the liquid crystal display apparatus 100 is used in the ultrasound diagnostic apparatus 200.

### [Description of Embodiments]

A preferable embodiment of the present disclosure will be described below in detail with reference to the attached drawings. Note that, in the present specification and the drawings, constituent elements that have substantially the same functional configuration are assigned the same reference numerals, and thus, redundant description thereof is omitted.

Note that the description will be given in the following order.
1. Overview
2. Embodiment
2.1. Configuration
2.2. Details of Arrangement of Components
2.3. Method for Fixing Components
2.4. Use of Condensing Film 120 That Partially Have Reflection Structure
3. Configuration of Ultrasound Diagnostic Apparatus 200
4. Summary

### <1. Overview>

Firstly, an overview of the present disclosure will be described.

Regarding a liquid crystal display, the value of retardation Δn·d, which is the product of the refractive-index anisotropy Δn of a liquid crystal material and the thickness d of a liquid crystal layer, becomes greater than 0 due to light that is incident on a liquid crystal panel from a direction other than the normal direction of a liquid crystal panel, resulting in degradation of the black brightness and degradation of the contrast ratio. Also, regarding the pair of polarizing plates that form a crossed Nicol prism in the liquid crystal panel as well, viewing angle dependency occurs with respect to light incident from a direction other than the normal direction, resulting in degradation of the black brightness and degradation of the contrast ratio.

As a countermeasure to this event, techniques for adjusting the light incident on the liquid crystal panel in the normal direction of the display surface (or a direction close to the normal direction) have been actively developed. These techniques are broadly divided into techniques for adjusting the propagation direction of light on the light source side and techniques for adjusting the propagation direction of light on the downstream side of the light source.

The former techniques include, for example, a technique of forming the shape of a light guide plate used in an edge light type light source so as to be parallel with the normal direction of the liquid crystal panel. However, in this technique, high accuracy is required in optical positioning between a point light source or a linear light source and the light guide plate, and adjustment of the brightness distribution in the display is difficult, and accordingly the mass productivity of the liquid crystal display apparatus is low.

The latter techniques include, for example, a technique of propagating light toward the normal direction of the liquid crystal panel by condensing light emitted from a light source using a prism sheet and a sheet in which a fine louver is incorporated, as in the aforementioned PTL 1. With this technique, propagation of light toward the normal direction of the liquid crystal panel may be realized more readily, and therefore the mass productivity of the liquid crystal display apparatus is high.

However, there have been cases where the contrast ratio is not sufficiently improved by the technique disclosed in PTL 1 or the like. For example, there are cases where the contrast ratio is not sufficiently improved due to the louver being not appropriately arranged with respect to a crossed Nicol prism formed by the pair of polarizing plates arranged respectively on a light source side and a display surface side of a liquid crystal panel, or the viewing angle improving sheet.

The discloser of this application came to create the technique of the present application in view of the foregoing circumstances. A liquid crystal display apparatus according to the present disclosure includes: a liquid crystal panel including a pair of substrates arranged in an opposing manner, and a liquid crystal layer sandwiched between the pair of substrates; a pair of polarizing plates arranged on a light source side and a display surface side, respectively, of the liquid crystal panel, and forming a crossed Nicol prism; a condensing film (first optical element) in which a light-blocking layer and a light-transmitting layer are alternately and continuously arranged in a planar direction, the first optical element being arranged between the polarizing plate on the light source side and the light source; and a viewing angle control film (second optical element) having a surface scattering function and arranged on the polarizing plate on the display surface side.

In the liquid crystal display apparatus according to the present disclosure, the condensing film is arranged such that a major axis of the light-blocking layer is oriented in a direction determined based on at least one of the crossed Nicol prism and the viewing angle control film. More specifically, the condensing film is arranged such that the major axis of the light-blocking layer tilts with respect to one of the two polarizing axes of the crossed Nicol prism, or the condensing film is arranged such that the angle formed by the major axis of the light-blocking layer and the major axis of the viewing angle control film takes a predetermined value.

The present disclosure can realize a high contrast ratio and a high black grade due to the condensing film being arranged such that the major axis of the light-blocking layer tilts with respect to one of the two polarizing axes of the crossed Nicol prism. Also, the present disclosure can realize a high contrast ratio due to the condensing film being arranged such that the angle formed by the major axis of the light-blocking layer and the major axis of the viewing angle control film takes a predetermined value. Hereinafter, an embodiment of the present disclosure will be described in detail. Note that the aforementioned "black grade" refers to the quality of black in the display that is determined based on the viewing angle dependency of black brightness. It can be said that the lower the viewing angle dependency of black brightness is (i.e. the less the black brightness greatly changes depending on the viewing angle), the higher the black grade is.

### <2. Embodiment>

Subsequently, a liquid crystal display apparatus 100 according to an embodiment of the present disclosure will be described. Here, the liquid crystal display apparatus 100 according to the present embodiment may be used in, for example, ultrasound diagnostic apparatuses, which are required to have a high contrast ratio. For example, in a B mode (Brightness mode), which is a basic mode of the ultrasound diagnostic apparatuses, the contrast ratio is important since an internal structure of a biological tissue is expressed only with the shades of black and white. As mentioned above, there are cases where organic EL displays are mounted in the ultrasound diagnostic apparatuses in order to realize a high contrast ratio, but the manufacturing costs of the organic EL displays tend to be relatively high, and thus the manufacturing costs of the entire ultrasound diagnostic apparatuses tend to increase. In contrast, as a result of the liquid crystal display apparatus 100 according to the present embodiment being used in the ultrasound diagnostic apparatuses, a high contrast ratio can be realized while suppressing the manufacturing costs of the entire ultrasound diagnostic apparatuses. Note that target apparatuses and systems in which the liquid crystal display apparatus 100 according to the present embodiment is to be used are not specifically limited. In other words, the present disclosure may be applied to any apparatuses and systems that have a liquid crystal display.

### (2.1. Configuration)

First, a configuration of the entire liquid crystal display apparatus 100 according to the present embodiment will be described with reference to Figs. 1 and 2. As shown in Fig. 1, the liquid crystal display apparatus 100 includes a backlight unit 140, a condensing film 120 that makes light emitted from the backlight unit 140 into light that propagates in the normal direction (or a direction close to the normal direction) of a liquid crystal panel 110, the liquid crystal panel 110 that displays a video using the light, and a viewing angle control film 130 that controls the viewing angle by scattering light emitted from the liquid crystal panel 110.

### (Backlight Unit 140)

The backlight unit 140 includes a backlight 141 and a DBEF (Dual Brightness Enhancement Film) 142, as shown in Fig. 2. The backlight 141 is a member that functions as a light source for emitting light to be used to display a video. The member that constitutes the backlight 141 may be any member capable of emitting any kind of light, and is not specifically limited. For example, the backlight 141 may be constituted by light-emitting diodes (LEDs), fluorescent tubes (e.g. CCLFs: Cold Cathode Fluorescent Lamps), or the like. The DBEF 142 is an optical element capable of improving the efficiency of use of light by recycling light, and capable of improving the brightness. In other words, the DBEF 142 can reduce power consumption under the same brightness conditions compared with the case where it is not used, and can reduce the amount of light of the backlight 141, the number of LEDs or the number of fluorescent tubes, and so on. Note that although the present embodiment will describe, as an example, the case where the DBEF 142 is used, the invention is not necessarily limited thereto, and any optical elements capable of recycling light other than the DBEF 142 may be applicable.

### (Condensing Film 120)

The condensing film 120 is an optical element (first optical element) capable of making incident light into light that propagates in the normal direction (or a direction close to the normal direction) of the liquid crystal panel 110 due to light-blocking layers 10 and light-transmitting layers 11 being alternately and continuously arranged in a planar direction. Fig. 3 is a conceptual diagram of the condensing film 120. As shown in Fig. 3, in the condensing film 120, the light-blocking layers 10 that block light and the light-transmitting layers 11 that transmit light are alternately and continuously arranged in the planar direction. Note that, as shown in Fig. 3, the direction in which the light-blocking layers 10 and the light-transmitting layers 11 are stacked in the planar direction of the condensing film 120 will be referred to as a "minor-axis direction", and a direction perpendicular to the minor-axis direction will be referred to as a "major-axis direction".

The condensing film 120 is basically arranged between the liquid crystal panel 110 and the backlight unit 140. Light that propagates in the normal direction of the liquid crystal panel 110 and light that propagates in a direction close to the normal direction are transmitted through the light-transmitting layers 11 without being absorbed by the light-blocking layers 10. On the other hand, light that propagates while greatly tilting with respect to the normal direction and light that is incident on some of the light-blocking layers 10, of the light that propagates in the normal direction (or a direction close to the normal direction) are absorbed by the light-blocking layers 10 and are not transmitted through the light-transmitting layers 11. Thus, due to having the light-blocking layers 10 and the light-transmitting layers 11, the condensing film 120 can make incident light into light that propagates in the normal direction (or a direction close to the normal direction) of the liquid crystal panel 110.

Based on the above description, it should be noted that the major-axis direction of the light-blocking layers 10 can be said to be a "direction in which light is transmitted", and the minor-axis direction of the light-blocking layers 10 can be said to be a "direction in which light is not transmitted (a direction in which light is absorbed)". It should also be noted that the light-blocking layers 10 are also referred to as "louver layers" or "louvers".

Note that, as mentioned above, the condensing film 120 is basically arranged between the liquid crystal panel 110 and the backlight unit 140, but may alternately be arranged between the backlight 141 and the DBEF 142, for example. Although the effect of improving the contrast ratio can also be achieved by this configuration, the efficiency of light recycling in the backlight unit 140 is decreased by the light-blocking layers 10, and thus the brightness decreases. Accordingly, it is desirable that the condensing film 120 is arranged between the liquid crystal panel 110 and the backlight unit 140.

Also, the number of condensing films 120 provided in the liquid crystal display apparatus 100 is not specifically limited. For example, as shown in Fig. 2, two condensing films 120 (a condensing film 121 and a condensing film 122 in the diagram) may be provided. Thus, the light propagation direction is controlled more strictly, and therefore the contrast ratio further improves (note that the larger the number of condensing films 120 is, the lower the brightness is due to the influence of light loss caused by the light-blocking layers 10). More detailed installation modes of the condensing film 120 will be described later in detail.

### (Liquid Crystal Panel 110)

The liquid crystal panel 110 is an optical element that includes a pair of substrates arranged in an opposing manner, and a liquid crystal layer 115 sandwiched between the pair of substrates. As shown in Fig. 2, the liquid crystal panel 110 further includes a pair of polarizing plates (a polarizing plate 111 on the backlight unit 140 side and a polarizing plate 112 on the display surface side) that form a crossed Nicol prism on the backlight unit 140 side and the display surface side (Fig. 2 omits the pair of substrates and the liquid crystal layer 115 for convenience). It should be noted that "crossed Nicol" refer to a state where the polarizing axis (polarizing direction) of the polarizing plate 111 and the polarizing axis of the polarizing plate 112 are located substantially perpendicular to each other.

Here, the display mode of the liquid crystal display according to the present embodiment may be, for example, a VA (Vertical Alignment) mode, an IPS (In Plane Switching) mode, a TN (Twisted Nematic) mode, or the like, but is not limited thereto. The configuration of the liquid crystal panel 110 is flexibly changed in accordance with the display mode. For example, when the display mode is the VA mode, in a non-driving state where a driving voltage is not applied to transparent electrodes (not shown) formed on the inner side of respective oriented films of the pair of substrates of the liquid crystal panel 110, liquid crystal molecules in the liquid crystal layer 115 are oriented substantially perpendicular to the substrate surfaces and, as a result, the polarization state of the light that is transmitted through the liquid crystal panel 110 hardly changes. In other words, the liquid crystal display apparatus 100 realizes a black display when in the non-driving state. In contrast, in a driving state, the liquid crystal molecules tilt substantially parallel to the electrode substrate surfaces, and the light that is transmitted through the liquid crystal panel 110 changes the polarization state thereof due to the thus-tilted liquid crystal molecules. In other words, the liquid crystal display apparatus 100 realizes a white display when in the driving state. As to display modes other than the VA mode as well, processing for orienting and driving the liquid crystal molecules corresponding to the display modes or the like is performed.

### (Viewing Angle Control Film 130)

The viewing angle control film 130 is an optical element (second optical element) that has a surface scattering function and is arranged on the polarizing plate 112 on the display surface side of the liquid crystal panel 110. Fig. 4 is a conceptual diagram of the viewing angle control film 130. As shown in Fig. 4, the viewing angle control film 130 includes incident-side optical elements 12 and exit-side optical elements 13, which are formed into continuous recessed and protruding shapes with one or more axes arranged in the planar direction. The direction in which the recessed and protruding shapes are continuous will be referred to as a "minor-axis direction", and a direction perpendicular to the minor-axis direction will be referred to as a "major-axis direction". That is to say, it should be noted that the minor-axis direction of the viewing angle control film 130 can be said to be a "direction in which light scatters", and the major-axis direction of the viewing angle control film 130 can be said to be a "direction in which light does not scatter".

The viewing angle control film 130 can appropriately control the viewing angle due to the incident-side optical elements 12 and the exit-side optical elements 13 having different refractive indexes. For example, if the relationship holds in which the refractive index of the incident-side optical elements 12 < the refractive index of the exit-side optical elements 13, light incident on the viewing angle control film 130 is refracted in a direction moving away from the normal direction of the liquid crystal panel 110 at interfaces between the incident-side optical elements 12 and the exit-side optical elements 13. In other words, in this case, the viewing angle is expanded in the minor-axis direction of the viewing angle control film 130 (conversely, if the relationship holds in which the refractive index of the incident-side optical elements 12 > the refractive index of the exit-side optical elements 13, the viewing angle is reduced in the minor-axis direction). Thus, the viewing angle control film 130 can appropriately control the viewing angle using the optical elements with different refractive indexes and the shapes thereof. This allows the viewing angle to be appropriately controlled by the viewing angle control film 130 in the case where the liquid crystal display apparatus 100 is mounted in an automobile or the like, for example, and thus a video may be prevented from being reflected on a windshield or the like. In the following, a description will be given while taking, as an example, the case where the relationship holds in which the refractive index of the incident-side optical elements 12 < the refractive index of the exit-side optical elements 13 (i.e. the case where the viewing angle is expanded in the minor-axis direction of the viewing angle control film 130).

Note that the shapes of the optical elements included in the viewing angle control film 130 are not limited to the shapes shown in Fig. 4. For example, these optical elements may have shapes that partially include a curved face. Also, the viewing angle control film 130 may also have a configuration that enables the viewing angle to be expanded (or reduced) in a two-dimensional direction (e.g. a 0° azimuth direction and a 90° azimuth direction, etc.). Also, as mentioned above, the viewing angle control film 130 has a function of scattering light, but the "scattering" may be replaced with refracting, diffusing, dispersing, diverging, or the like, as appropriate.

### (2.2. Details of Arrangement of Components)

Subsequently, the details of the arrangement of the above-described components will be described. First, a description will be given, with reference to Figs. 5 to 8, of a difference in the viewing angle dependency of black brightness before and after the present disclosure is applied. Fig. 5 shows measured values of the viewing angle for black brightness before the present disclosure is applied. More specifically, Fig. 5 shows results of measuring the black brightness of the display in the azimuth range from 0° to 360°, and in the radiation angle (elevation angle) range from 0° to 90°. Note that, in this example, the polarizing axes of the crossed Nicol prism are set at 0° and 90°.

As shown in Fig. 5, it can be understood that the black brightness is relatively high around azimuths of 45°, 135°, 225°, and 315° (in other words, it can be understood that light leaks around these azimuths). That is to say, before the present disclosure is applied, a considerable degree of light leaks particularly between the polarizing axes of the crossed Nicol prism in the liquid crystal panel 110, and the black grade is likely to decrease.

For this reason, in the present embodiment, the condensing film 120 is arranged such that the major axis of the light-blocking layers 10 in the condensing film 120 tilts with respect to one of the two polarizing axes of the crossed Nicol prism. For example, one condensing film 121 is arranged such that the angle formed by the major axis of the light-blocking layers 10 and one of the two polarizing axes of the crossed Nicol prism is 45°, and furthermore, the other one condensing film 122 is arranged such that the major axis of the light-blocking layers 10 is perpendicular to that of the condensing film 121. Fig. 6 shows measured values of the viewing angle for black brightness after the present configuration is applied. Note that, in Fig. 6, since measured values that are about 0.1 [cd/m²] or less are densely distributed, the measured values are omitted for convenience (also see Fig. 7 together). That is to say, as a result of the present configuration being applied, light that leaked around azimuths of 45°, 135°, 225°, and 315° is absorbed by the condensing film 121 and the condensing film 122.

Fig. 7 shows, as an example, measured values of the black brightness at each radiation angle at azimuths of 45° and 225° (note that, in the example in Fig. 7, the radiation angle in the 225° azimuth direction is shown in the range from -90° to 0° for convenience). As shown in Fig. 7, after the configuration of the present disclosure is applied, the black brightness significantly decreases (which is particularly significant around radiation angles of 60° and -60°), and the difference in the black brightness between radiation angles is small compared with before the application of the configuration of the present disclosure. That is to say, the present disclosure can realize a high black grade and a high contrast ratio due to the condensing film 120 being arranged such that the major axis of the light-blocking layers 10 tilts with respect to one of the two polarizing axes of the crossed Nicol prism.

As mentioned above, in the present embodiment, the condensing film 120 is arranged such that the angle formed by the major axis (the axis in the direction in which light is transmitted) of the light-blocking layers 10 and the major axis (the axis perpendicular to the direction in which light scatters; i.e. the axis in the direction in which the viewing angle is not expanded) of the viewing angle control film 130 takes a predetermined value. For example, the closer the predetermined value is to 0°, the more the contrast ratio improves due to the relationship between the light-condensing function of the condensing films 120 and the light-scattering function of the viewing angle control film 130. More specifically, the contrast ratio improves since the light-condensing direction of the condensing films 120 coincides with the scattering direction of the viewing angle control film 130.

Here, a description will be given, with reference to Fig. 8, of the relationship between the azimuth of the major axis of the light-blocking layers 10 in the condensing film 120, the black brightness, and the contrast ratio. Fig. 8 shows the black brightness and the contrast ratio at each azimuth of the major axis of the light-blocking layers 10 when the polarizing axes of the liquid crystal panel 110 are set at 0° and 90°, and the major axis of the viewing angle control film 130 is set at 90°.

As shown in Fig. 8, regarding the black brightness, favorable values are obtained in the range of the major axis of the light-blocking layers 10 of about 30° or larger and about 60° or smaller, and it can be said that this range of azimuth is a range in which the black grade is high (which is denoted as "high black-grade range" in the diagram). Meanwhile, regarding the contrast ratio, favorable values are obtained in the range of the major axis of the light-blocking layers 10 of about 0° or larger and about 45° or smaller, and it can be said that this range of azimuth is a range in which the contrast ratio is high (which is denoted as "high contrast-ratio range" in the diagram). Based on these results, the azimuth of the major axis of the light-blocking layers 10 may be flexibly determined in accordance with required quality. For example, the azimuth of the major axis of the light-blocking layers 10 may be set to about 30° or more and about 45° or less when both a high black grade and a high contrast ratio are required, and the azimuth of the major axis of the light-blocking layers 10 may be set to about 0° when a high contrast ratio is required rather than a high black grade.

Here, a description will be given, with reference to Figs. 9 to 17, of variations of the installation modes of the liquid crystal panel 110, the condensing film 120, and the viewing angle control film 130. Note that the angles shown in Figs. 9 to 17 indicate the azimuths of the axes of the components (e.g. the major axis of the light-blocking layers 10 in the condensing film 120, etc.).

Figs. 9 to 12 show variations of the installation modes of the liquid crystal panel 110, the condensing film 120, and the viewing angle control film 130 when the display mode of the liquid crystal panel 110 is the VA mode or the IPS mode (note that it is assumed that the polarizing axes of the liquid crystal panel 110 are at azimuths of 0° and 90° in the VA mode and the IPS mode).

For example, when the polarizing axes of the liquid crystal panel 110 are set at azimuths of 0° and 90°, and the major axis of the viewing angle control film 130 is set at an azimuth of 90° as shown in Fig. 9, the major axes of the respective light-blocking layers 10 in the two condensing films 120 may be set at azimuths of 45° and 135°. Since the angles formed by the major axes of the respective light-blocking layers 10 in the two condensing films 120 and the two polarizing axes are 45°, a high black grade and a high contrast ratio are realized. In addition, a high contrast ratio is also realized in that the angles formed by the major axes of the respective light-blocking layers 10 in the two condensing films 120 and the major axis of the viewing angle control film 130 are 45°.

Also, as shown in Fig. 10, when the polarizing axes of the liquid crystal panel 110 are set at azimuths of 0° and 90°, and the major axis of the viewing angle control film 130 is set at an azimuth of 90°, the major axis of the light-blocking layers 10 of one condensing film 120 may be set at an azimuth of 90°. A high contrast ratio is realized since the angle formed by the major axis of the light-blocking layers 10 in the condensing film 120 and the major axis of the viewing angle control film 130 is 0°. Also, as a result of one condensing film 120 being used, a higher brightness is realized than in the case where two or more condensing films 120 are used.

Also, as shown in Fig. 11, when the polarizing axes of the liquid crystal panel 110 are set at azimuths of 0° and 90°, and the respective major axes of two viewing angle control films 130 are set at azimuths of 45° and 135°, the major axes of the respective light-blocking layers 10 in two condensing films 120 may be set at azimuths of 45° and 135°. Since the angles formed by the major axes of the respective light-blocking layers 10 in the two condensing films 120 and the two polarizing axes are 45°, a high black grade and a high contrast ratio are realized. In addition, a high contrast ratio is also realized in that the angles formed by the major axes of the respective light-blocking layers 10 in the two condensing films 120 and the major axes of the two viewing angle control films 130 are 0°. Furthermore, the field of view is expanded in two directions (azimuths of 45° and 135°) by the two viewing angle control films 130.

Also, as shown in Fig. 12, when the polarizing axes of the liquid crystal panel 110 are set at azimuths of 0° and 90°, and the respective major axes of two viewing angle control films 130 are set at azimuths of 0° and 90°, the major axes of the respective light-blocking layers 10 in two condensing films 120 may be set at azimuths of 45° and 135°. Since the angles formed by the major axes of the respective light-blocking layers 10 in the two condensing films 120 and the two polarizing axes are 45°, a high black grade and a high contrast ratio are realized. In addition, a high contrast ratio is also realized in that the angles formed by the major axes of the respective light-blocking layers 10 in the two condensing films 120 and the major axes of the two viewing angle control films 130 are 45°. Furthermore, the field of view is expanded in two directions (azimuths of 0° and 90°) by the two viewing angle control films 130.

Figs. 13 to 15 show variations of the installation modes of the liquid crystal panel 110, the condensing film 120, and the viewing angle control film 130 when the display mode of the liquid crystal panel 110 is the TN mode (note that it is assumed that the polarizing axes of the liquid crystal panel 110 are at azimuths of 45° and 135° in the TN mode).

For example, as shown in Fig. 13, when the polarizing axes of the liquid crystal panel 110 are set at azimuths of 45° and 135°, and the major axis of the viewing angle control film 130 is set at an azimuth of 90°, the major axes of the respective light-blocking layers 10 in the two condensing films 120 may be set at azimuths of 0° and 90°. Since the angles formed by the major axes of the respective light-blocking layers 10 in the two condensing films 120 and the two polarizing axes are 45°, a high black grade and a high contrast ratio are realized. In addition, a high contrast ratio is also realized in that the angle formed by the major axis of the light-blocking layers 10 in one of the condensing films 120 and the major axis of the viewing angle control film 130 is 0°.

Also, as shown in Fig. 14, when the polarizing axes of the liquid crystal panel 110 are set at azimuths of 45° and 135°, and the major axis of the viewing angle control film 130 is set at an azimuth of 90°, the major axes of the respective light-blocking layers 10 in two condensing films 120 may be set at azimuths of 60° and 120°. Since the angles formed by the major axes of the respective light-blocking layers 10 in the two condensing films 120 and the major axis of the viewing angle control film 130 are 30°, a high contrast ratio is realized.

Also, as shown in Fig. 15, when the polarizing axes of the liquid crystal panel 110 are set at azimuths of 45° and 135°, and the major axis of the viewing angle control film 130 is set at an azimuth of 90°, the major axis of the light-blocking layers 10 in one condensing film 120 may be set at an azimuth of 90°. Since the angles formed by the major axis of the light-blocking layers 10 in the condensing film 120 and the two polarizing axes are 45°, a high black grade and a high contrast ratio are realized. In addition, a high contrast ratio is also realized in that the angle formed by the major axis of the light-blocking layers 10 in the condensing film 120 and the major axis of the viewing angle control film 130 is 0°. Also, as a result of one condensing film 120 being used, a higher brightness is realized than in the case where two or more condensing films 120 are used.

Figs. 16 and 17 show variations of the installation modes of the liquid crystal panel 110, the condensing film 120, and the viewing angle control film 130 when the viewing angle is expanded in a specific direction other than 0° and 90° (note that although the description is given while taking, as an example, the case where the liquid crystal panel 110 whose display mode is the VA mode or the IPS mode is used, this need not be the case). Regarding in-vehicle devices, game machine, mobile phones, smartphones, or the like, the direction in which the viewing angle is expanded is determined in accordance with the direction in which the user visually recognizes the display.

For example, as shown in Fig. 16, when the polarizing axes of the liquid crystal panel 110 are set at azimuths of 0° and 90°, and the major axis of the viewing angle control film 130 is set at an azimuth of 135°, the major axes of the respective light-blocking layers 10 in two condensing films 120 may be set at azimuths of 45° and 135°. The viewing angle is expanded in the directions at azimuths of 45° and 225° due to the major axis of the viewing angle control film 130 being set at an azimuth of 135°. In addition, since the angles formed by the major axes of the respective light-blocking layers 10 in the two condensing films 120 and the two polarizing axes are 45°, a high black grade and a high contrast ratio are realized. Furthermore, a high contrast ratio is also realized in that the angle formed by the major axis of the light-blocking layers 10 in one of the condensing films 120 and the major axis of the viewing angle control film 130 is 0°.

Also, as shown in Fig. 17, when the polarizing axes of the liquid crystal panel 110 are set at azimuths of 0° and 90°, and the major axis of the viewing angle control film 130 is set at an azimuth of 135°, the major axes of the respective light-blocking layers 10 in two condensing films 120 may be set at azimuths of 120° and 150°. The viewing angle is expanded in the directions at azimuths of 45° and 225°, similarly to Fig. 16, due to the major axis of the viewing angle control film 130 being set at an azimuth of 135°. In addition, since the angles formed by the major axes of the respective light-blocking layers 10 in the two condensing films 120 and one of the two polarizing axes are 30°, a high black grade and a high contrast ratio are realized. Furthermore, a high contrast ratio is also realized in that the angles formed by the major axes of the respective light-blocking layers 10 in the two condensing films 120 and the major axis of the viewing angle control film 130 are 15°.

Note that the installation modes of the liquid crystal panel 110, the condensing film 120, and the viewing angle control film 130 are not limited to the examples in Figs. 9 to 17. For example, the direction in which the viewing angle is expanded can be flexibly changed. Although it has been described that the azimuths of the polarizing axes of the liquid crystal panel 110 are 0° and 90° in the VA mode and the IPS mode, and the azimuths of the polarizing axes of the liquid crystal panel 110 are 45° and 135° in the TN mode, the azimuths are not necessarily limited to these azimuths. The numbers of condensing films 120 and the viewing angle control films 130 are not specifically limited.

### (2.3. Method for Fixing Components)

The details of the arrangement of the components have been described above. Subsequently, a method for fixing the components will be described. Particularly, a method for fixing the liquid crystal panel 110, the condensing film 120, and the backlight unit 140 will be described.

As described above, the liquid crystal display apparatus 100 according to the present embodiment includes the condensing film 120, but the brightness of the liquid crystal display apparatus 100 decreases due to the condensing film 120 being used since the condensing film 120 has the light-blocking layers 10. Therefore, it is desirable that the liquid crystal panel 110 (strictly speaking, the polarizing plate 111), the condensing film 120, and the backlight unit 140 (strictly speaking, the DBEF 142) are adhered (optically adhered) to each other by means of an optically transparent member. As a result, the number of reflecting surfaces decreased, and thus Fresnel loss can be avoided. Here, the optically transparent member may be an OCA (Optical Clear Adhesive) or the like, for example, but is not necessarily limited thereto.

However, if the liquid crystal panel 110 (strictly speaking, the polarizing plate 111) and the condensing film 120 are adhered (optically adhered) to each other by the optically transparent member, there may be cases where moiré, light-blocking layers 10 showing (a phenomenon in which the light-blocking layers 10 becomes visible to a user), warping of the liquid crystal panel 110 (and a Newton ring caused by the warping of the liquid crystal panel 110), or the like occurs due to the optically transparent member, and image quality deteriorates.

In this case, as shown in Fig. 18, films with a low-reflection structure called moth eye structure may be arranged such that the structures oppose each other, on the opposing surfaces of the liquid crystal panel 110 (strictly speaking, the polarizing plate 111) and the condensing film 120 (strictly speaking, the condensing film 122). In the following description, these films will be referred to as "moth eye films" (third optical element). In the example in Fig. 18, a moth eye film 160 is provided on the surface of the condensing film 122, and a moth eye film 161 is provided on the surface of the polarizing plate 111. The moth eye structure is a low-reflection structure that continuously varies its refractive index with respect to incident light in the thickness direction using protrusions that are smaller than the visible light wavelengths, thereby eliminating a discontinuity interface in refractive index and realizing low reflection. Accordingly, light reflection becomes extremely small at the interfaces between the moth eye films 160 and 161 and an air layer 170, and thus, the same degree of image quality as that in the case where adhesion (optical adhesion) is performed can be realized with the occurrence of the aforementioned problem suppressed. Note that other optical elements with an antireflection function may be used in place of the moth eye films. In Fig. 18, the condensing film 121 and the DBEF 142 are adhered to each other by an OCA 150, and the condensing film 121 and the condensing film 122 are adhered to each other by an OCA 151.

### (2.4. Use of Condensing Film 120 That Partially Have Reflection Structure)

A method for fixing the components has been described above. Subsequently, the use of the condensing film 120 that partially has a reflection structure will be described.

The liquid crystal display apparatus 100 according to the present embodiment includes the condensing film 120, but the efficiency of use of light decreases since light is absorbed by the light-blocking layers 10 in the condensing film 120. For example, as shown in A in Fig. 19, light that is incident on the light-blocking layers 10 in the condensing film 120 from the backlight unit 140 (light denoted by (1) in the diagram) and light that is reflected off the polarizing plate 111 and incident on the light-blocking layers 10 (light denoted by (2) in the diagram) are absorbed by the light-blocking layers 10, and are therefore not used for indication on the display.

For this reason, in the present embodiment, light-blocking layers 10a whose light-incident surface partially has a reflection structure may be used. More specifically, as shown in B in Fig. 19, light-blocking layers 10a may be used that have a reflection structure on at least one of the light-incident surfaces (on the backlight unit 140 side and the liquid crystal panel 110 side). For example, the reflection structure of the light-blocking layers 10a may be realized as a result of white ink containing metal (such as silver), titanium oxide, or the like being applied onto light-incident surfaces of the light-blocking layers 10a. Thus, light that is incident on the light-blocking layers 10a in the condensing film 120 from the backlight unit 140 (light denoted by (3) in the diagram) and light that is reflected off the polarizing plate 111 and incident on the light-blocking layers 10a (light denoted as (4) in the diagram) are reflected off a part of each light-blocking layer 10a, and thus the brightness of the liquid crystal display apparatus 100 improves due to light recycling. Note that the method for realizing the reflection structure of the light-blocking layers 10a is not limited to the above.

### <3. Configuration of Ultrasound Diagnostic Apparatus 200>

The liquid crystal display apparatus 100 according to an embodiment of the present disclosure has been described above. Subsequently, a description will be given, with reference to Fig. 20, of a configuration of an ultrasound diagnostic apparatus 200 in the case where the above-described liquid crystal display apparatus 100 is used in the ultrasound diagnostic apparatus 200.

As shown in Fig. 20, the ultrasound diagnostic apparatus 200 includes a probe 210, a transmission circuit 220, a reception circuit 230, a B-mode processing circuit 240, a Doppler processing circuit 250, a processing unit 260, a liquid crystal display 270, and a storage device 280.

### (Probe 210)

The probe 210 is configured to irradiate an object with an ultrasound signal and receive the ultrasound signal reflected off the object. More specifically, the probe 210 has a plurality of ultrasonic oscillators, and the plurality of ultrasonic oscillators generate ultrasound signals based on a drive signal supplied from the transmission circuit 220. The probe 210 transmits a beam-shaped ultrasound signal to the inside of the object by converging the ultrasound signals generated from the plurality of ultrasonic oscillators, and further receives the ultrasound signal reflected off the object and converts the received ultrasound signal to an electrical signal.

### (Transmission Circuit 220)

The transmission circuit 220 has a trigger generation circuit, a transmission delay circuit, a pulsar circuit, and so on, and supplies a drive signal to the probe 210. The pulsar circuit repeatedly generates, at a predetermined rate frequency, a rate pulse for forming an ultrasound signal. The transmission delay circuit gives each rate pulse generated by the pulsar circuit a delay time for each piezoelectric oscillator that is required to converge the ultrasound signal generated from the probe 210 into a beam shape and determine transmission directivity. Also, the trigger generation circuit applies a drive pulse signal to the probe 210 at a timing based on the rate pulse. The delay circuit adjusts the direction of transmission from a piezoelectric oscillator surface in any manner by varying the delay time given to each rate pulse.

### (Reception Circuit 230)

The reception circuit 230 has an amplifier circuit, an A/D converter, an adder, and so on, receives the electrical signal received by the probe 210, performs various kinds of processing on this electrical signal, and generates reflected wave data. The amplifier circuit amplifies the electrical signal for each channel and performs gain correction processing. The A/D converter performs A/D conversion on the gain-corrected electrical signal, and gives the converted digital data a delay time required to determine reception directionality. The adder performs addition processing on the electrical signal processed by the A/D converter and generates reflected wave data. Reflection components from a direction corresponding to the reception directivity of the electrical signal are enhanced through the addition processing performed by the adder.

### (B-mode Processing Circuit 240)

The B-mode processing circuit 240 performs logarithmic amplification, envelope detection processing, and so on, on the reflected wave data received from the reception circuit 230, and generates data (B-mode data) whose signal intensity is expressed as brightness.

### (Doppler Processing Circuit 250)

The Doppler processing circuit 250 performs frequency analysis on the reflected wave data received from the reception circuit 230, extracts blood flow, tissue, and contrast agent echo components obtained due to the Doppler effect, and generates data obtained by extracting moving body information, such as average velocity, dispersion, and power, regarding multiple points (Doppler data).

### (Processing Unit 260)

The processing unit 260 is configured to generate image information (ultrasound image information) based on the ultrasound signal reflected off the object. More specifically, the processing unit 260 generates B-mode image information that expresses the intensity of the ultrasound signal reflected off the object as brightness, based on the B-mode data generated by the B-mode processing circuit 240. Also, the processing unit 260 generates color Doppler image information as an average velocity image, a dispersion image, a power image, or a combination image thereof that expresses the moving body information, based on the Doppler data generated by the Doppler processing circuit 250. The processing unit 260 enables the liquid crystal display 270 to display such image information by supplying the generated image information to the liquid crystal display 270. Note that the image information generated by the processing unit 260 is not limited to the above. Also, the content of processing performed by the processing unit 260 is not limited to the above. For example, the processing unit 260 also performs processing to control operation of the probe 210 via the transmission circuit 220.

### (Liquid Crystal Display 270)

The liquid crystal display 270 is configured to display the image information generated by the processing unit 260, and is realized by the above-described liquid crystal display apparatus 100. That is to say, the liquid crystal display 270 includes the liquid crystal panel 110 that includes the pair of substrates (the substrate 113 and the substrate 114) arranged in an opposing manner and the liquid crystal layer 115 sandwiched between these two substrates, the pair of polarizing plates (the polarizing plate 111 and the polarizing plate 112) arranged on the light source side and the display surface side, respectively, of the liquid crystal panel 110 and forming a crossed Nicol prism, the condensing film 120 (first optical element) in which the light-blocking layers 10 and the light-transmitting layers 11 are alternately and continuously arranged in a planar direction, the condensing film 120 being arranged between the polarizing plate 111 on the light source side and a light source, and the viewing angle control film 130 (second optical element) with a surface scattering function arranged on the polarizing plate 112 on the display surface side. In the liquid crystal display 270, the condensing film 120 is arranged such that the major axis of the light-blocking layers 10 is oriented in a direction determined based on at least one of the crossed Nicol prism and the viewing angle control film 130. More specifically, the condensing film 120 is arranged such that the major axis of the light-blocking layers 10 tilts with respect to one of the two polarizing axes of the crossed Nicol prism, or the condensing film 120 is arranged such that the angle formed by the major axis of the light-blocking layers 10 and the major axis of the viewing angle control film 130 takes a predetermined value.

As described above, organic EL displays have been conventionally used in ultrasound diagnostic apparatuses, whereas a high contrast ratio can be realized while suppressing the manufacturing costs of the entire ultrasound diagnostic apparatus 200 as a result of the liquid crystal display 270 realized with the liquid crystal display apparatus 100 being used in the ultrasound diagnostic apparatus 200, as shown in Fig. 20. Note that the liquid crystal display 270 may also have components other than the components included in the liquid crystal display apparatus 100.

### (Storage Device 280)

The storage device 280 is configured to store various kinds of information. For example, the storage device 280 can store the image information generated by the processing unit 260 and store various programs and various parameters used in the processing performed by the processing unit 260. Note that the information stored in the storage device 280 is not limited to such information.

An example configuration of the ultrasound diagnostic apparatus 200 has been described above. Note that the above configuration described with reference to Fig. 20 is merely an example, and the configuration of the ultrasound diagnostic apparatus 200 is not limited to such an example. For example, the ultrasound diagnostic apparatus 200 need not necessarily include all of the components shown in Fig. 20. Also, the configuration of the ultrasound diagnostic apparatus 200 can be flexibly modified in accordance with specifications and operations.

### <4. Summary>

As described above, in the liquid crystal display apparatus 100 according to the present disclosure, the condensing film 120 is arranged such that the major axis of the light-blocking layers 10 is oriented in a direction determined based on at least one of the crossed Nicol prism and the viewing angle control film 130. More specifically, the condensing film 120 is arranged such that the major axis of the light-blocking layers 10 tilts with respect to one of the two polarizing axes of the crossed Nicol prism, or the condensing film 120 is arranged such that the angle formed by the major axis of the light-blocking layers 10 and the major axis of the viewing angle control film 130 takes a predetermined value.

The present disclosure can realize a high contrast ratio and a high black grade due to the condensing film 120 being arranged such that the major axis of the light-blocking layers 10 tilts with respect to one of the two polarizing axes of the crossed Nicol prism. Also, the present disclosure can realize a high contrast ratio due to the condensing film 120 being arranged such that the angle formed by the major axis of the light-blocking layers 10 and the major axis of the viewing angle control film 130 takes a predetermined value.

Although a preferable embodiment of the present disclosure has been described above in detail with reference to the attached drawings, the technical scope of the present disclosure is not limited to such an example. It is apparent that a person having common knowledge in the technical field of the present disclosure may conceive various changes or modifications within the scope of the technical idea described in the claims, and it is understood that such changes and modifications also naturally pertain to the technical scope of the present disclosure.

The effect described in the present specification is merely descriptive or exemplary, and is not limited. That is to say, the technique according to the present disclosure may have other effects that are apparent to those skilled in the art from the description of the present specification, in addition to or in place of the above-described effect.

Note that the following configurations also pertain to the technical scope of the present disclosure.
(1) An ultrasound diagnostic apparatus including:
   a probe that irradiates an object with an ultrasound signal and receives an ultrasound signal reflected off the object;
   a processing unit that generates image information based on the reflected ultrasound signal; and
   a liquid crystal display that displays the image information, wherein
   the liquid crystal display includes:
      a liquid crystal panel including a pair of substrates arranged in an opposing manner, and a liquid crystal layer sandwiched between the pair of substrates;
      a pair of polarizing plates arranged on a light source side and a display surface side, respectively, of the liquid crystal panel, and forming a crossed Nicol prism;
      a first optical element in which a light-blocking layer and a light-transmitting layer are alternately and continuously arranged in a planar direction, the first optical element being arranged between the polarizing plate on the light source side and the light source; and
      a second optical element having a surface scattering function and arranged on the polarizing plate on the display surface side, and
   the first optical element is arranged such that a major axis of the light-blocking layer that is an axis extending in a direction in which light is transmitted is oriented in a direction determined based on at least one of the crossed Nicol prism and the second optical element.
(2) The ultrasound diagnostic apparatus described in the item (1) above, wherein the first optical element is arranged such that the major axis of the light-blocking layer tilts with respect to one of the two polarizing axes of the crossed Nicol prism.
(3) The ultrasound diagnostic apparatus described in the item (2) above,
   wherein the first optical element is arranged such that an angle formed by the major axis of the light-blocking layer and one of the two polarizing axes of the crossed Nicol prism is included in a range of 30°or larger and 60° or smaller.
(4) The ultrasound diagnostic apparatus according to any one of the items (1) to (3) above, wherein
   the first optical element is arranged such that an angle formed by the major axis of the light-blocking layer and a major axis of the second optical element that is an axis perpendicular to a direction in which light scatters takes a predetermined value.
(5) The ultrasound diagnostic apparatus described in the item (4) above, wherein the predetermined value is included in a range of 0° or larger and 45° or smaller.
(6) The ultrasound diagnostic apparatus according to any one of the items (1) to (5) above, wherein
   a third optical element having a low-reflection structure is further arranged on each of opposing surfaces of the polarizing plate on the light source side and of the first optical element.
(7) The ultrasound diagnostic apparatus described in the item (6) above, wherein the low-reflection structure is a moth eye structure.
(8) The ultrasound diagnostic apparatus according to any one of the items (1) to (7) above, wherein
   a light-incident surface of the light-blocking layer partially has a reflection structure.
(9) The ultrasound diagnostic apparatus according to any one of the items (1) to (8) above, further including
   the light source that emits light toward the first optical element.
(10) A liquid crystal display apparatus including:
   a liquid crystal panel including a pair of substrates arranged in an opposing manner, and a liquid crystal layer sandwiched between the pair of substrates;
   a pair of polarizing plates arranged on a light source side and a display surface side, respectively, of the liquid crystal panel, and forming a crossed Nicol prism;
   a first optical element in which a light-blocking layer and a light-transmitting layer are alternately and continuously arranged in a planar direction, the first optical element being arranged between the polarizing plate on the light source side and the light source; and
   a second optical element having a surface scattering function and arranged on the polarizing plate on the display surface side, wherein
   the first optical element is arranged such that a major axis of the light-blocking layer that is an axis extending in a direction in which light is transmitted is oriented in a direction determined based on at least one of the crossed Nicol prism and the second optical element.
(11) The liquid crystal display apparatus according to the item (10) above, wherein
   the first optical element is arranged such that the major axis of the light-blocking layer tilts with respect to one of the two polarizing axes of the crossed Nicol prism.
(12) The liquid crystal display apparatus according to the item (11) above, wherein
   the first optical element is arranged such that an angle formed by the major axis of the light-blocking layer and one of the two polarizing axes of the crossed Nicol prism is included in a range of 30°or larger and 60° or smaller.
(13) The liquid crystal display apparatus according to any one of the items (10) to (12) above, wherein
   the first optical element is arranged such that an angle formed by the major axis of the light-blocking layer and a major axis of the second optical element that is an axis perpendicular to a direction in which light scatters takes a predetermined value.
(14) The liquid crystal display apparatus according to the item (13) above, wherein the predetermined value is included in a range of 0° or larger and 45° or smaller.
(15) The liquid crystal display apparatus according to any one of the items (10) to (14) above, wherein
   a third optical element having a low-reflection structure is further arranged on each of opposing surfaces of the polarizing plate on the light source side and of the first optical element.
(16) The liquid crystal display apparatus according to the above item (15), wherein the low-reflection structure is a moth eye structure.
(17) The liquid crystal display apparatus according to any one of the items (10) to (16) above, wherein
   a light-incident surface of the light-blocking layer partially has a reflection structure.
(18) The liquid crystal display apparatus according to any one of the items (10) to (17) above, further including
   the light source that emits light toward the first optical element.

### [Reference Signs List]

100 Liquid crystal display apparatus
110 Liquid crystal panel
111, 112 Polarizing plate
113, 114 Substrate
115 Liquid crystal layer
120 Condensing film
130 Viewing angle control film
140 Backlight unit
141 Backlight
142 DBEF
150, 151 OCA
160, 161 Moth eye film
170 Air layer

## Claims

1. An ultrasound diagnostic apparatus comprising:
a probe that irradiates an object with an ultrasound signal and receives an ultrasound signal reflected off the object;
a processing unit that generates image information based on the reflected ultrasound signal; and
a liquid crystal display that displays the image information, wherein
the liquid crystal display includes:
a liquid crystal panel including a pair of substrates arranged in an opposing manner, and a liquid crystal layer sandwiched between the pair of substrates;
a pair of polarizing plates arranged on a light source side and a display surface side, respectively, of the liquid crystal panel, and forming a crossed Nicol prism;
a first optical element in which a light-blocking layer and a light-transmitting layer are alternately and continuously arranged in a planar direction, the first optical element being arranged between the polarizing plate on the light source side and the light source; and
a second optical element having a surface scattering function and arranged on the polarizing plate on the display surface side, and
the first optical element is arranged such that a major axis of the light-blocking layer that is an axis extending in a direction in which light is transmitted is oriented in a direction determined based on at least one of the crossed Nicol prism and the second optical element.

2. The ultrasound diagnostic apparatus according to claim 1, wherein
the first optical element is arranged such that the major axis of the light-blocking layer tilts with respect to one of the two polarizing axes of the crossed Nicol prism.

3. The ultrasound diagnostic apparatus according to claim 2, wherein
the first optical element is arranged such that an angle formed by the major axis of the light-blocking layer and one of the two polarizing axes of the crossed Nicol prism is included in a range of 30°or larger and 60° or smaller.

4. The ultrasound diagnostic apparatus according to claim 1, wherein
the first optical element is arranged such that an angle formed by the major axis of the light-blocking layer and a major axis of the second optical element that is an axis perpendicular to a direction in which light scatters takes a predetermined value.

5. The ultrasound diagnostic apparatus according to claim 4, wherein
the predetermined value is included in a range of 0° or larger and 45° or smaller.

6. The ultrasound diagnostic apparatus according to claim 1, wherein
a third optical element having a low-reflection structure is further arranged on each of opposing surfaces of the polarizing plate on the light source side and of the first optical element.

7. The ultrasound diagnostic apparatus according to claim 6, wherein
the low-reflection structure is a moth eye structure.

8. The ultrasound diagnostic apparatus according to claim 1, wherein
a light-incident surface of the light-blocking layer partially has a reflection structure.

9. The ultrasound diagnostic apparatus according to claim 1, further comprising the light source that emits light toward the first optical element.

10. A liquid crystal display apparatus comprising:
a liquid crystal panel including a pair of substrates arranged in an opposing manner, and a liquid crystal layer sandwiched between the pair of substrates;
a pair of polarizing plates arranged on a light source side and a display surface side, respectively, of the liquid crystal panel, and forming crossed Nicol prism;
a first optical element in which a light-blocking layer and a light-transmitting layer are alternately and continuously arranged in a planar direction, the first optical element being arranged between the polarizing plate on the light source side and the light source; and
a second optical element having a surface scattering function and arranged on the polarizing plate on the display surface side, wherein
the first optical element is arranged such that a major axis of the light-blocking layer that is an axis extending in a direction in which light is transmitted is oriented in a direction determined based on at least one of the crossed Nicol prism and the second optical element.

11. The liquid crystal display apparatus according to claim 10, wherein
the first optical element is arranged such that the major axis of the light-blocking layer tilts with respect to one of the two polarizing axes of the crossed Nicol prism.

12. The liquid crystal display apparatus according to claim 11, wherein
the first optical element is arranged such that an angle formed by the major axis of the light-blocking layer and one of the two polarizing axes of the crossed Nicol prism is included in a range of 30°or larger and 60° or smaller.

13. The liquid crystal display apparatus according to claim 10, wherein
the first optical element is arranged such that an angle formed by the major axis of the light-blocking layer and a major axis of the second optical element that is an axis perpendicular to a direction in which light scatters takes a predetermined value.

14. The liquid crystal display apparatus according to claim 13, wherein
the predetermined value is included in a range of 0° or larger and 45° or smaller.

15. The liquid crystal display apparatus according to claim 10, wherein
a third optical elements having a low-reflection structure is further arranged on each of opposing surfaces of the polarizing plate on the light source side and of the first optical element.

16. The liquid crystal display apparatus according to claim 15, wherein
the low-reflection structure is a moth eye structure.

17. The liquid crystal display apparatus according to claim 10, wherein
a light-incident surface of the light-blocking layer partially has a reflection structure.

18. The liquid crystal display apparatus according to claim 10, further comprising the light source that emits light toward the first optical element.
